# EUROPEAN PATENT APPLICATION

(11) **EP 4 563 159 A1**
(43) Date of publication of application: **04.06.2025**
(21) Application number: 23213600.2
(22) Date of filing: 01.12.2023
(51) Int. Cl.: A61K 39/12, A61P 31/14

(54) **MUCOSAL VACCINATION VIA AN IGA FEEDBACK LOOP**

(71) Applicant: Forschungszentrum Borstel, Leibniz Lungenzentrum, 23845 Borstel (DE)
(72) Inventor: FREY, Andreas, 23816 Groß Niendorf (DE); FREY, Barbara, 23816 Groß Niendorf (DE); RAMAKER, Katrin, 22397 Hamburg (DE); RÖCKENDORF, Niels, 22941 Bargteheide (DE)
(74) Representative: Moré, Solveig Helga

(57) **Abstract**

The present invention relates to the field of immunology, in particular, to mucosal vaccination. It provides a vaccine comprising a conjugate of an agent capable of specifically binding to an IgA or IgM, and an antigen. Said vaccine is advantageous for use in mucosal vaccination of a subject against said antigen, as it exploits an IgA feedback loop. The invention also provides methods for mucosal vaccination comprising administration of the vaccine of the invention to a subject. Furthermore, improved IgA binding peptides are disclosed.

## Description

The present invention relates to the field of immunology, in particular, to mucosal vaccination. It provides a vaccine comprising a conjugate of an agent capable of specifically binding to IgA or IgM, and an antigen. Said vaccine is advantageous for use in mucosal vaccination of a subject against said antigen, as it exploits an IgA feedback loop. The invention also provides methods for mucosal vaccination comprising administration of the vaccine of the invention to a subject. Furthermore, improved IgA binding peptides are disclosed.

The mucosal surfaces represent the largest outer surface of the body and are constantly exposed to pathogens and foreign antigens. To protect these surfaces, the mucosal immune system secretes immunoglobulins of class A (secretory IgA, slgA) onto the mucosal surfaces. At mucosal sites, IgA is produced in the lamina propria by local plasma cells, mainly as dimeric molecules (dimeric IgA; dlgA) containing a joining J chain. It can bind to the polymeric Ig receptor (plgR) that is expressed on the basolateral membrane of epithelial cells. Subsequently, it is transported through the epithelial cells and released into the lumen as slgA. slgA further comprises the secretory component (SC), a fragment of the plgR, that protects the immunoglobulin from degradation.

IgA, such as slgA, can intercept pathogens and foreign antigens outside the body, cross-link them and detain them in mucus, thereby preventing or reducing infection. Consequently, induction of relevant, pathogen-specific IgA is a highly desired goal for vaccinations. It is a severe drawback of conventionally delivered systemic immunizations that, in general, they do not lead to mucosal immune responses. Secretory IgA are predominantly induced when the respective antigen is taken up at the mucosal surfaces and transported via the epithelium to the underlying mucosa-associated lymphoid tissue. At those immune-inductive sites the antigen must then be presented in the context of suitable proinflammatory or danger signals. Due to these complex requirements for a successful mucosal immunization, the development of mucosal vaccines has been hampered with only little progress over the last decades, although the benefit from vaccination via the mucosal route is being acknowledged (Holmgren & Svennerholm, 2012; Baker et al. 2022; Topol & Iwasaki, 2022).

Worldwide a limited number of mucosal vaccines exist which have been approved for use in humans (Holmgren & Svennerholm 2012; Correa et al. 2021; Lavelle & Ward, 2022). These vaccines are applied either orally or as nasal sprays or nose drops. Targets of the orally delivered vaccines are the viral pathogens poliovirus and rotavirus, and the bacterial pathogens *Vibrio cholerae* and *Salmonella typhimurium.* Nasal spray-delivered vaccines are directed against influenza virus or corona virus. The efficacy of mucosal vaccines has been demonstrated most impressively with the two antiviral oral vaccines: mass vaccinations with oral polio vaccine within the Global Polio Eradication Initiative has led to a nearly complete eradication of poliomyelitis. National vaccination programs with oral rotavirus vaccines have decreased the mortality due to diarrheal diseases by 25-50% in the respective countries (Troeger et al., 2018; Tate & Parashar, 2014). Both, the oral polio as well as the rotavirus vaccine, are live vaccines employing an attenuated form of the virus. They therefore bear a certain risk to build revertants with regained virulence. Specifically in the case of polio vaccination, the spread of vaccine-derived polio viruses gives cause for concern, and revertants may lead to severe disease outbreaks in vaccinees or their non-vaccinated contact persons (Burns et al., 2014; Roberts 2018). Hence, although the health benefit of the oral vaccinations for society as a whole is undisputable, side effects caused by the live vaccines remain a serious draw back.

Nasally applied vaccines which are mainly aimed at protecting against respiratory infections are still not very successful. One nasal spray vaccine against influenza is approved for use in children in Europe and for children and adults in the USA; again, this live attenuated influenza vaccine (LAIV) suffers from side effects which manifest in various, often severe influenza-like symptoms.

An interesting approach to mucosal vaccination using an IgA feedback loop was suggested by scientists around Blaise Corthésy in the 1990s (Corthésy et al., 1996; Crottet et al., 1999; Rochereau et al., 2013). They used externally produced recombinant slgA molecules into which an antigen of interest was cloned or to which it was coupled, together with an adjuvant for mucosal vaccination in animals. A slgA response against the antigen of interest was successfully induced. However, a production of recombinant slgA for use as a mucosal vaccine is expensive, and thus not economically viable on a large scale. Fransen et al. (2015) also showed that the presence of bacteria-binding IgAs (be they innate or antigen-driven) favors the induction of antigen-specific IgAs through a positive feedback loop.

Others exploited the capability of albumin to be transcytosed across the mucosal epithelium through interactions with neonatal Fc receptors for albumin hitchhiking, wherein antigens of interest were modified with an albumin-binding lipid tail (Liu et al., 2014; Hartwell et al., 2022), or through interactions with plgR (US 2022112276 A1).

Further, mucosal vaccines which are not based on live/attenuated pathogens or viral/bacterial vectors usually require the addition of adjuvants or immunostimulatory additives in order to induce an immune response (Corthésy et al., 2018; Szoka 2022). There are clinical studies for a few formulations (e.g., oral application of liposomes with *Streptococcus mutans* glycosyltransferase, pneumococcus vaccine with different recombinant proteins, *Helicobacter pylori* urease), but the effect was always weak and none of the formulations has so far been approved for human use. There is at present no effective adjuvant that can support mucosal vaccination in humans with non-pathogen-based vaccines.

More recent approaches include vector-based vaccines for mucosal vaccination, wherein a genetically engineered carrier virus, e.g., adenovirus or mumps virus, expresses antigens of interest, e.g., SARS-CoV-2 surface antigens. Such vaccines have been successful in animal studies, but have led to conflicting results in clinical studies. There are admissions for one vaccine of this type for human use in China and in India, respectively.

In light of this, the inventors addressed the problem of providing improved compositions for inducing a mucosal immune response and for mucosal vaccination. This problem is solved by the present invention, in particular, as laid out in the appended claims.

The invention provides a vaccine comprising a conjugate of
a) an agent capable of specifically binding to an immunoglobulin, wherein the immunoglobulin is IgA or IgM, and
b) an antigen or a nucleic acid, e.g., RNA, encoding an antigen

for use in mucosal vaccination of a subject against said antigen,
wherein the vaccine does not comprise IgA or IgM, wherein the vaccine preferably does not comprise an adjuvant.

The invention is based on the fact that both IgA and IgM can be taken up into the mucosa by retrotranscytosis (also called reverse transcytosis, Rochereau et al., 2021). Throughout the invention, the immunoglobulin to which the agent can specifically bind preferably is IgA, which is secreted by the mucosa in large amounts. The immunoglobulin can also be IgM, e.g., if the subject is IgA deficient.

IgA can be non-secretory IgA, which can also be transcytosed, but it preferably is slgA. The IgA can, in primates, be IgA1 or IgA2. IgA2 is more stable to gastrointestinal tract conditions. In primates, IgA preferably is IgA2. slgA typically comprises dimeric IgA and SC. Binding can be binding to any part of the IgA, e.g., to the light chain, the heavy chain, the joining chain or to the SC.

IgM preferably is slgM. Binding can be binding to any part of the IgM, e.g., to the light chain, the heavy chain, the joining chain or to the SC.

An agent capable of specifically binding to IgA is also designated an IgA binder herein, and an agent capable of specifically binding to IgM is designated an IgM binder.

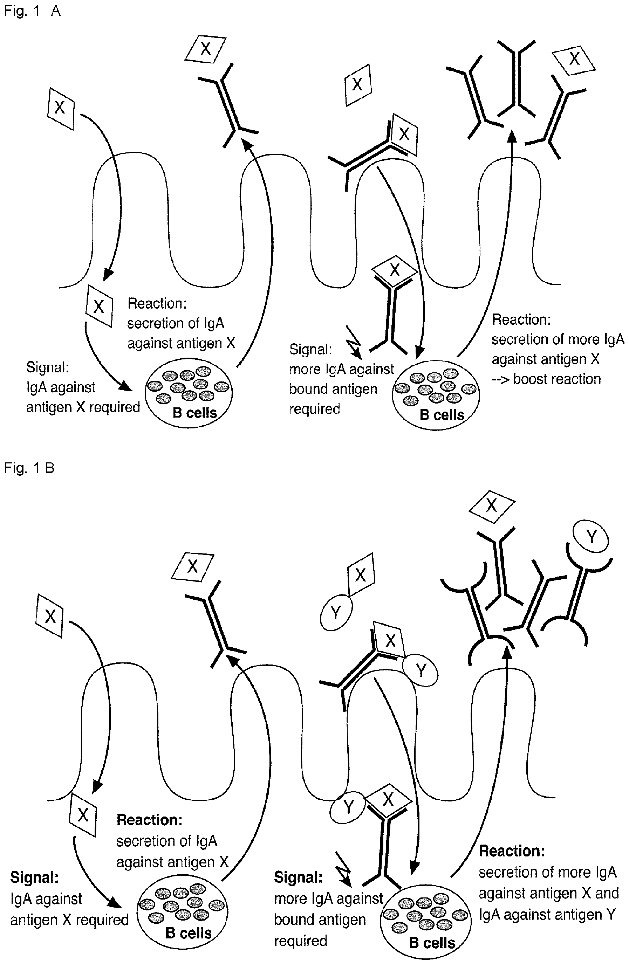

The present invention addresses two essential problems of mucosal vaccinations: the transepi-thelial transport of the vaccine to the submucosal immune-inductive areas, as well as the provision of immunostimulatory signals enabling priming of an IgA-based immune reaction at the mucosa. The invention exploits an IgA feedback loop. This regulatory mechanism of the mucosal immune system takes account of the necessity for production or increased production of slgA in the presence of a target antigen and stimulates the specific IgA secretion in case of need, e.g., for a quick response upon re-infection with a previously encountered pathogen. For this, a fraction of the slgA patrolling the lumen is permanently reinternalized via the mucosal epithelial tissue and transported to the submucosal lymphoid tissue. If these IgA are loaded with an antigen, this is considered an immunostimulatory signal by the immune system, and production of further slgA to this antigen is induced (Fig. 1A, top).

This feedback loop has been previously exploited (Corthésy et al., 1996; Crottet et al., 1999; Rochereau et al., 2013; Rochereau et al., 2014; Rochereau et al., 2015; Rochereau et al., 2016; Rochereau et al., 2021; Rochereau et al., 2021, Fransen et al., 2015, EP 0 839 915 A1, WO 98/20141 A1) using externally produced recombinant slgA molecules into which an antigen of interest was cloned or to which it was coupled, together with an adjuvant for experimental mucosal vaccination in animal experiments. In contrast, in the novel mucosal vaccination of the invention, the use of externally produced, recombinant or synthetic slgA is avoided, which renders the vaccine of the invention more commercially viable. Instead, the IgA feedback loop, or the corresponding IgM feedback loop is employed using endogenous immunoglobulins, i.e., immunoglobulins produced by the subject that is to be vaccinated her- or himself, e.g., permanently secreted IgA. The vaccine of the invention thus does not comprise IgA. Preferably, the vaccine of the invention also does not comprise an Fc part of IgA or a secretory component. It also does not comprise IgM or an Fc part of IgM. Optionally, it also does not comprise any other Fc part of an immunoglobulin. It is not required that the vaccine of the invention comprises a component capable of binding to FcRn.

Albumin, ovalbumin, cholera toxin B, fimbriae or other antigens from pathogens can be used as specific IgA binding agents binding to the paratope of IgA in the context of the invention. However, it is not limited to these, and alternative IgA or IgM binders can be used. Thus, in one embodiment, the vaccine also does not comprise albumin, ovalbumin, cholera toxin B, fimbriae or other antigens from pathogens as IgA binding agents.

After application of the vaccine to the mucosa, e.g., nasally, the conjugate is bound to slgA or slgM secreted into the lumen (e.g., the airways or the gastrointestinal tract) via the agent capable of specifically binding to IgA or IgM. The antigen of component b) of the conjugate, i.e., the antigen to which the subject is to be vaccinated, is thus also associated with IgA or IgM, and they are together transcytosed to the immuno-inductive subepithelial regions of the mucosa. There, a mucosal immune response, in particular, an IgA response to the antigen is induced (Fig. 1B).

Advantageously, the novel vaccine can be flexibly adapted for vaccination to different antigens, i.e., it can be used for vaccination against different pathogens or cancers. The antigen for vaccination can be coupled to the agent capable of binding to IgA or IgM and different vaccines can thus be prepared. An agent capable of binding to IgA or IgM that has been admitted for human use can be easily adapted for different vaccinations. Also, variations of the antigen, e.g., for adapting the antigen to different pathogen variants, such as different SARS-CoV-2 variants, is easily possible, and shall lead to an improved immune response. The vaccination of the invention can also be used for vaccination against a different pathogen or cancer following a first or subsequent vaccination using the vaccine of the invention.

Further, a pre-existing immunity to the agent capable of binding to IgA or IgM, e.g., from earlier vaccinations, is not problematic (e.g., as with vector approaches), but in contrast improves efficiency of subsequent vaccinations with the same agent capable of binding to IgA or IgM. The vaccine of the invention can thus advantageously be used in vaccination schemes involving priming and boosting, optionally, several times, e.g., with priming and 1, 2, 3, 4, 5, 6 or more boosts.

Further, advantageously, the vaccine does not require, and preferably, does not comprise an adjuvant and thus avoids problems that can be caused by adjuvants, such as toxicity, potential non-specific induction of immune responses that may lead to autoimmunity or other adverse reactions. In the context of the invention, the vaccine also does not comprise live or attenuated bacteria or a viral vector. The inventors found that this is not required for priming an efficient immune response or avoiding tolerance.

Preferably, throughout the invention, the conjugate of the vaccine used in the invention is a covalent conjugate. For example, the antigen may be covalently coupled to the agent. If the IgA binder or IgM binder is a polypeptide, the conjugate may be a fusion protein, but, advantageously, that is not required. Chemical coupling, e.g., via lysine or cysteine residues of the antigen and agent, is a preferred option for conjugation.

Alternatively, the conjugate may be a non-covalent conjugate. For this, the affinity must be rather high, so that in one embodiment for non-covalently coupled conjugates, the conjugate comprises biotin-streptavidin or biotin-avidin or a non-lgA antibody that can specifically bind to the antigen via its paratope or a fragment of said antibody that comprises an Fv region, preferably, an scFv. However, this introduces a further immunogen into the vaccine, which can be disadvantageous. The conjugate may also comprise a multi (e.g., bi) -specific binder, e.g., it may comprise an scFc capable of specifically binding to IgA and an scFv capable of specifically binding to the antigen.

The conjugate may thus also comprise a linker between the agent and the antigen, e.g., a peptidic linker. The length of the linker may be, e.g., 1-50 aa, such as 2-20 aa or 3-10 aa. In one specific embodiment, the linker may comprise a polyethylene glycol moiety of a length of 2 to 40, e.g., 10-20, (C₂H₄O)-units.

Specific binding of the agent capable of binding to IgA or IgM does not exclude binding to other proteins, e.g., with different domains, or binding to IgG. The agent can also be capable of binding to both IgA and IgM. The affinity of the agent to the immunoglobulin can be, e.g., in the range of at most 5 mM, at most 4 µM, at most 3 µM, 2 µM, preferably, at most 1 µM, e.g., at most 500 nM, at most 200 nM, at most 100 nM, at most 50 nM, at most 20 nM, at most 10 nM, at most 5 nM, at most 2 nM or at most 1 nM. For specific antibody-antigen binding, an affinity in the lower nM down to pM range can be found, while a generic binder can have an affinity of at most 5 µM, preferably, at most 1 µM, e.g., at most 100 nM or at most 10 nM.

Affinities can be measured by surface plasmon resonance biosensors, such as Biacore (e.g., as disclosed in Yang et al. (2016) Anal. Biochem. 508:78-96), preferably, affinity is measurable as EC₅₀ in a solid-phase binding assay as described in the examples. Advantageously, throughout the invention, the EC₅₀ of the agent capable of specifically binding to an immunoglobulin, e.g., the IgA-binding peptide, is 5 µM or less as determined in a solid-phase binding assay.

The affinity determined in a solid-phase binding assay is often higher than the affinity found in solution (e.g., determined by isothermal titration calorimetry as in Kirley & Norman (2022) Biochem Biophys Reports 32:101354). Thus, optionally, the affinity mentioned is also reached in solution.

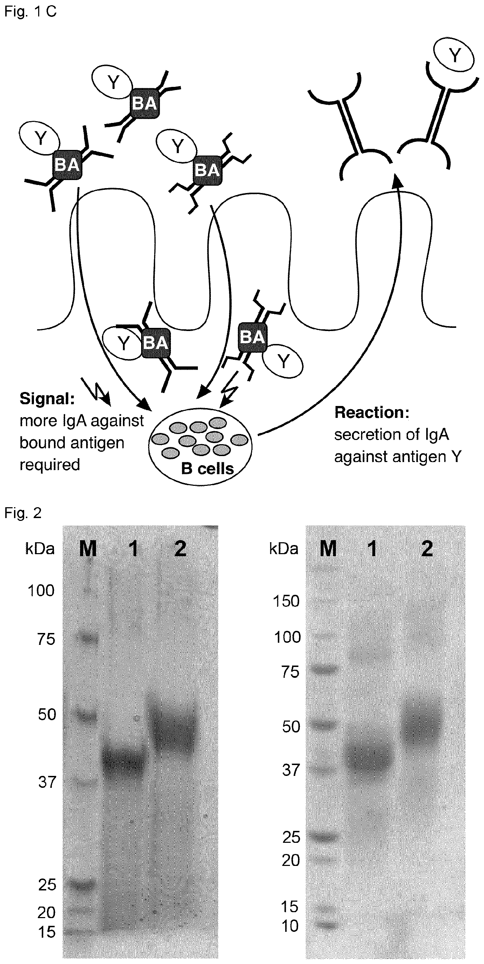

The vaccine for use of the invention can be used in two basic variants that differ in the function of the agent capable of specifically binding to an immunoglobulin, wherein the immunoglobulin is IgA or IgM (cf. Fig. 1B & 1C).
1) The agent capable of specifically binding to the immunoglobulin can be capable of specifically binding to the paratope of pre-existing IgA or IgM in the subject. In other words, it can be the antigen recognized by such pre-existing immunoglobulins, or it can comprise the epitope recognized by such pre-existing immunoglobulins. The agent thus has a high affinity for IgA or IgM. Further, both IgM and IgA and the retrograde transcytosis addressed by them can be targeted at the same time. There are antigens capable of specifically binding to the paratope of pre-existing IgAs or IgMs in a large proportion of subjects in the human population, e.g., the majority of the population in Germany have IgA specifically binding to SARS-CoV-2 spike protein. There are other wide-spread mucosal immunities, e.g., for rhi-noviruses specific for respiratory diseases, or for gastrointestinal pathogens, e.g., in particular in African populations, gastrointestinal pathogens spreading through water contaminations, or for rotavirus or polio (where oral vaccination is still used).
2) The agent capable of specifically binding to the immunoglobulin can be an agent that is capable of specifically binding to the immunoglobulin independently from the paratope of the immunoglobulin (i.e., the specific antigen-binding site), e.g., it can bind to a constant region, e.g., an Fc part of the IgA or IgM, to the joining chain (J-chain) or to the secretory component (SC). While, typically, the affinity of such agents is lower than affinity between the par-atopes of an immunoglobulin and its specific antigen, this is more than compensated for by the quantity of immunoglobulins available. An agent that is capable of specifically binding to the immunoglobulin independently from the paratope can bind to all IgM or IgA (or slgM or slgA, preferably, slgA), respectively, not only to a proportion of them that specifically binds to one antigen. Advantageously, efficiency of the vaccination of the invention thus also does not have to rely on any specific pre-existing immunity that may or may not be present in a large part of the population or in the subject that is to be vaccinated. Therefore, this embodiment is more generally applicable.

If the agent is capable of specifically binding to the immunoglobulin independently from the paratope of the immunoglobulin, e.g., to a constant part of the immunoglobulin, preferably, to a constant part of slgA, the agent can be
i) an IgA binder comprising an IgA binding peptide.

IgA binding peptides are known in the art, e.g., fragments of members of the M protein family, such as the Sir22 protein of *Streptococcus pyogenes, e.g.,* as disclosed in Johnsson et al. (1999) J. Biol. Chem. 274:14521 or WO 2000/63383A1. In one embodiment, the IgA-binding peptide has a sequence as disclosed and claimed in WO 2000/63383A1, e.g. SEQ ID NO: 3.

Preferred IgA binding peptides are e.g., disclosed by Ito et al. in WO 2011/148952 A1, or WO 2013/081037 A1 or in Hatanaka et al., 2012. In one embodiment, the peptide has a sequence as disclosed and claimed in WO 2011/148952 A1 or WO 2013/081037 A1 or described in Hatanaka et al.2012, e.g., a peptide which comprises an amino acid sequence consisting of 12 to 18 amino acid residues represented by the following formula I and is capable of binding to human IgA:
(X)₁₋₃-C-(X)₈₋₁₀-C-(X)₁₋₃
wherein each X independently represents an arbitrary amino acid other than cysteine, and C represents a cysteine residue. (X)₁₋₃ in this context means that 1-3 amino acids X may be present, and (X)₈₋₁₀ means that 8-10 amino acids X can be present. An arbitrary amino acid typically is a naturally occurring amino acid. It can also be, e.g., ornithine.

Such preferred IgA binding peptides may comprise SEQ ID NO: 4, consisting of
X1-X2-X3-C-L-X4-Y-X5-X6-X7-G-X8-X9-V-C-X10-X11-X12
wherein each X (i.e., X1-X12) independently represents an arbitrary amino acid residue other than cysteine and, optionally, X6 and/or X7 can alternatively be deleted; C represents a cysteine residue; L represents a leucine residue; Y represents a tyrosine residue; G represents a glycine residue; and V represents a valine residue. In a preferred embodiment, X6 and X7 are absent. Preferably, in SEQ ID NO: 4, the 1st to 18th amino acid residues counted from the N terminus in the case that the number of amino acid residues of the peptide is regarded as 18 amino acid residues, are respectively

| | |
|---|---|
| 1st amino acid residue X1 | = Q, H, K, R, S, or P, |
| 2nd amino acid residue X2 | = M, K, R, L, V, A, or D, |
| 3rd amino acid residue X3 | = R, L, M, or V, |
| 4th amino acid residue | = C, |
| 5th amino acid residue | = L, |
| 6th amino acid residue X4 | = S, H, Q, T, K, R, N, or A, |
| 7th amino acid residue | = Y, |
| 8th amino acid residue X5 | = K or R, |
| 9th amino acid residue X6 | = an arbitrary amino acid residue other than C or deleted, |
| 10th amino acid residue X7 | = an arbitrary amino acid residue other than C or deleted, |
| 11th amino acid residue | = G, |
| 12th amino acid residue X8 | = R, S, T, or K, |
| 13th amino acid residue X9 | = R, M, K, E, N, or P, |
| 14th amino acid residue | = V, |
| 15th amino acid residue | = C, |
| 16th amino acid residue X10 | = L, F, V, or I, |
| 17th amino acid residue X11 | = W, L, R, E, T, S, Q, P, or A, and |
| 18th amino acid residue X12 | = L, I, Y, A, or V. |

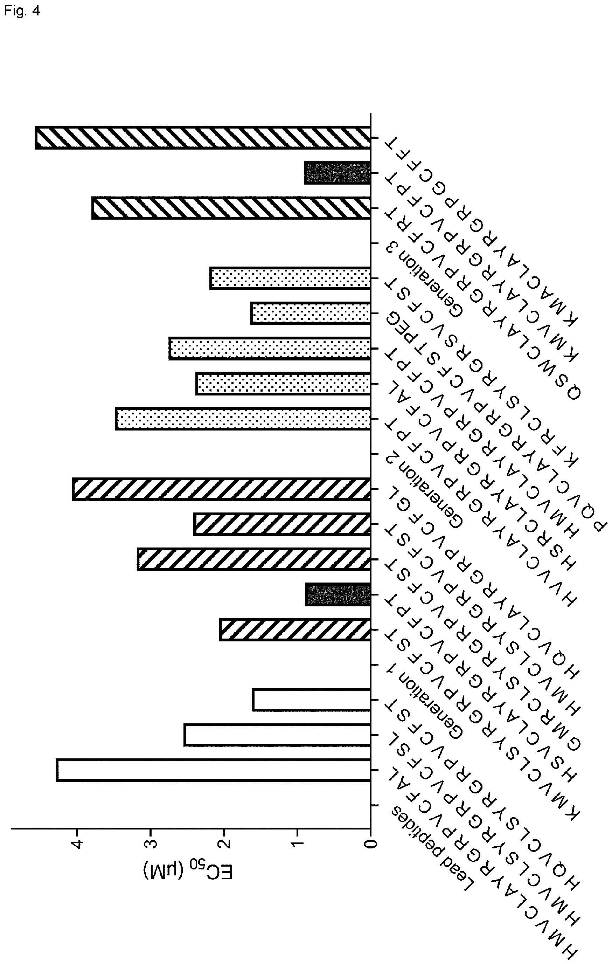

Examples of known IgA-binding peptides are, e.g., SEQ ID NO: 5-31. Peptides having SEQ ID NO: 29, 30 and 31 have particularly good binding affinities to IgA. Thus, they were used for comparison in the experiment underlying Fig. 4.

IgA binding peptides, e.g., as provided by Ito et al. may preferably be cyclic peptides. The length of the cyclic peptides preferably is 12-25 aa, preferably, 14-18 aa.

The present inventors have shown that affinities to IgA can be further improved by a molecular evolution process. Advantageous IgA binding peptides are 16mer peptides which share the consensus sequence SEQ ID NO: 32. SEQ ID NO: 32 is X1-X2-X3-C-L-X4-Y-X5-G-X6-X7-V-C-X8-X9-T
wherein each X independently represents an arbitrary amino acid residue other than cysteine; C represents a cysteine residue; L represents a leucine residue; Y represents a tyrosine residue; G represents a glycine residue; and V represents a valine residue. T represents a threonine residue. Preferably, X2 is M, S, F, V or Q. Further, in a preferred embodiment, X9 is P. Peptides of SEQ ID NO: 32 wherein X9 is P are novel over the state of the art. Most preferably, X9 is P and X2 is M, S, F, V or Q.

Preferred IgA binding peptides that can be used in the conjugate used in the invention have SEQ ID NO: 33, the consensus sequence of top peptides identified by the inventors during several courses of molecular evolution:
X1-X2-X3-C-L-X4-Y-R-G-R-X5-V-C-F-X6-X7
wherein the amino acids are

| | |
|---|---|
| 1st amino acid residue X1 | = H, K, Q, G or P, |
| 2nd amino acid residue X2 | = M, S, F, V or Q, |
| 3^{rd} amino acid residue X3 | = V or R, A, W (preferably, V), |
| 4th amino acid residue | = C, |
| 5th amino acid residue | = L, |
| 6th amino acid residue X4 | = A or S (preferably, A), |
| 7th amino acid residue | = Y, |
| 8th amino acid residue | = R, |
| 9th amino acid residue | = G, |
| 10th amino acid residue | = R, |
| 11th amino acid residue X5 | = P or S (preferably, P), |
| 12th amino acid residue | = V or G (preferably, V), |
| 13th amino acid residue | = C, |
| 14th amino acid residue | = F, |
| 15th amino acid residue X6 | = P, F, A, G or S (preferably, P) and |
| 16th amino acid residue X7 | = T or L (preferably, T) |

Preferably, X7 is T. Peptides of SEQ ID NO: 33 wherein X6 is P and X7 is T are novel over the state of the art and have a good binding affinity to IgA.

A preferred peptide of SEQ ID NO. 33 further has SEQ ID NO: 34, the consensus sequence of the top four peptides identified by the inventors:
X1-X2-X3-C-L-X4-Y-R-G-R-X5-V-C-F-X6-T
wherein the amino acids are

| | |
|---|---|
| 1st amino acid residue X1 | = H, K or P, |
| 2nd amino acid residue X2 | = M, S, F or Q, |
| 3rd amino acid residue X3 | = V or R (preferably, V), |
| 4th amino acid residue | = C, |
| 5th amino acid residue | = L, |
| 6th amino acid residue X4 | = A or S (preferably, A), |
| 7th amino acid residue | = Y, |
| 8th amino acid residue | = R, |
| 9th amino acid residue | = G, |
| 10th amino acid residue | = R, |
| 11th amino acid residue X5 | = P or S (preferably, P), |
| 12th amino acid residue | = V, |
| 13th amino acid residue | = C, |
| 14th amino acid residue | = F, |
| 15th amino acid residue X6 | = P or S (preferably, P) and |
| 16th amino acid residue | = T |

Peptides of SEQ ID NO: 34 wherein X6 is P are novel over the state of the art and have a very good binding affinity to IgA.

The inventors have shown that, advantageously, peptides of any of the consensus sequences disclosed herein have an IgA binding affinity, as measurable in an IgA binding assay, e.g., as disclosed herein, of at most 5 µM, preferably, of at most 4 µM, at most 3µM, at most 2µM, at most 1.5 µM or, most preferably, of at most 1 µM.

Particularly preferred IgA binding peptides, having the best identified binding affinities to IgA (EC₅₀ about 0.9 mM), have SEQ ID NO: 35 or SEQ ID NO: 36. SEQ ID NO: 37 to 47 also have advantageous binding affinities to IgA that make them particularly suitable for use in the conjugate of the invention. Peptides of SEQ ID NO: 35-47 were identified as the best binding peptides in their respective generation of molecular evolution (data showing a comparison of these peptides with state of the art peptides are shown in Fig. 4).

Of note, IgA binding peptides of SEQ ID NO: 32, 33 or 34, wherein, in each of those sequences, the 15^{th} amino acid residue is P and the 16^{th} amino acid residue is T, or any of SEQ ID NO: 35-47 have been newly identified as IgA binding peptides by the inventors as such. The invention thus also provides these peptides, compositions comprising them, e.g., pharmaceutical or diagnostic compositions comprising any of these peptides, or a combination thereof. These peptides can also be used for IgA binding in different contexts, e.g., in a method for isolating IgA, e.g., comprising contacting an IgA-containing solution to a solid support with bound IgA-binding peptides, or for targeting an active agent to an IgA-presenting cell, such as an IgA-presenting lymphoma cell, e.g., in treatment of cancer.
ii) an IgA binder comprising an anti-lgA antibody or consisting of a fragment of said antibody that comprises an Fv region. The IgA-binding antibody fragment comprises an IgA-binding paratope. Said IgA binder is not of IgA or IgM isotype. It can be a monoclonal or a polyclonal antibody, preferably, a monoclonal antibody. Optimally, it does not comprise an Fc region, which is not required, and which would furthermore increase the length and thus, the costs of production as well as antigenicity. Preferably, the IgA binder in this embodiment is an scFv, i.e, an anti-lgA scFv.
iii) an IgA receptor selected from the group comprising polymeric Ig receptor (plg receptor, involved in epithelial transport of IgA and/or IgM), myeloid specific IgA Fc receptor (FcαRI, also designated CD89), Fcα/µR (CD351), asialoglycoprotein receptor, M-cell IgA receptor and transferrin receptor (CD71) or a fragment of said IgA receptor capable of binding the Fc part of IgA.
iv) a small molecule having up to 800 Da. A small molecule typically is an organic molecule. It is not a biopolymer such as a protein, an oligosaccharide, polysaccharide or nucleic acid, but it can be a monomer thereof. Optionally, the small molecule is a nucleotide, a nucleoside, a nucleobase or an amino acid mimicking a nucleobase, preferably, a nucleotide. It has been shown that nucleotides can have a high affinity to antibodies, and can be used as ligands for antibodies. For example, nucleotides or nucleotide mimetics that bind to immunoglobulins, e.g., as disclosed in Rajagopalan et al. (1996) Proc. Natl. Acad. Sci 93:6019 or Alves et al. (2012) Anal. Chem. 84:7721, can be employed in the context of the invention.

The agent can also be protein A or protein G or derivatives thereof, e.g., as disclosed in WO 1984/001294 A1 or WO 2003/080655 A1, which can bind to IgA and/or IgM. The agent can also be an aptamer capable of binding to IgA or IgM independently from the paratope of the immunoglobulin.

The agent can also be an IgM binder selected from the group comprising protein A (e.g., as disclosed in US 9,156,892), FcµR, and Fcα/µR (CD351). It can also be an IgM binder comprising an anti-IgM antibody or consisting of a fragment of said antibody that comprises an Fv region. The IgM-binding antibody fragment comprises an IgM-binding paratope. Said IgM binder is not of IgA or IgM isotype. Optimally, it does not comprise an Fc region, which is not required, and which would furthermore increase the length and thus, the costs of production. Preferably, the IgM binder in this embodiment is an scFv, i.e, an anti-IgM scFv.

The agent, when bound to the immunoglobulin, does not prevent and preferably does not interfere with binding of the immunoglobulin (IgA or IgM) to Dectin-1, SIGLEC-5 or other receptors implicated in reverse transcytosis of slgA or slgM-based antigen complexes (Rochereau et al. 2013; Rochereau et al., 2021). The IgA binding agent can, e.g., bind to the Calpha3 of IgA.

The conjugate of the invention can also comprise two, three, four or more agents capable of binding to an immunoglobulin IgA or IgM, which can increase avidity. The two or more agents capable of binding to the immunoglobulin can be identical or they can be different, e.g., from different classes of agents capable of binding to an immunoglobulin independent of the paratope of the immunoglobulin as mentioned above. For example, the conjugate of the invention can comprise two IgA agents capable of specifically binding to the immunoglobulin independently from the paratope of the immunoglobulin, e.g., an IgA binding peptide and a small molecule such as a nucleotide. These can be linked to each other or to the antigen by a spacer.

As stated, alternatively, the agent can be capable of specifically binding to the paratope of pre-existing IgA or IgM in the subject. In this case, the agent may e.g., comprise a biopolymer, e.g., a polypeptide, comprising at least one B-cell epitope, e.g., of a common mucosal pathogen, preferably, the whole polypeptide, wherein the polypeptide may be selected from the group comprising a SARS-CoV-2 spike antigen, an influenza HA, a rhinovirus antigen, a *Streptococcus pneumoniae* antigen, a rotavirus antigen, and a salmonella antigen. Alternatively, the agent may be selected from the group of antigens of commensal bacterial species selected from the group comprising Lactobacillus, Bifidobacterium, Bacteroides, e.g., Bacteroides fragilis and Escherichia, e.g., *E. coli.* For example, the agent can be an oligosaccharide or polysaccharide of a commensal species such as Bacteroides. IgA antibodies against glycan structures of these widely spread commensal bacteria have been shown to be important for colonisation of the gut by these bacteria.

In a preferred embodiment, the vaccine is for use in vaccinating a human subject. It is clear to the skilled person, that in this case, the immunoglobulin to which the agent binds is human, e.g., human slgA. If alternatively, the subject is a mouse, said immunoglobulin is murine. If the subject is a pig, the immunoglobulin is a pig immunoglobulin, and so on. The subject can also be another mammal, such as a cow, a horse, a pig, a sheep, a goat, a camel, a cat, a dog, a mouse, a rat, a rabbit, or guinea pig. Typically, the mucosal vaccination is a preventive vaccination.

In the vaccine of the invention, the conjugate comprises an antigen or a nucleic acid, e.g., RNA, encoding the same. Preferably the antigen is directly contained in the conjugate. In this case, the antigen in the conjugate comprises at least one B-cell epitope, i.e., a region that can be bound by the paratope of an antibody. B cell epitopes may be linear, but they can also be conformational epitopes. The B-cell epitope typically is an epitope of a surface biopolymer of a pathogen that is targeted by the vaccination. Typically, the antigen comprises several B-cell epitopes, optionally, a complete surface biopolymer of a pathogen targeted by the vaccination or at least the extracellular part thereof (or the part that is accessible to antibodies on the surface of a pathogen, e.g., a virus). Preferably, the antigen is a polypeptide antigen, which can optionally be glycosylated. The antigen optimally also comprises at least one T cell epitope, i.e., an epitope that may be presented on an MHC I or II molecule of a cell of the subject, typically, a human HLA molecule. As expression of different MHC/HLA alleles varies throughout the population, the antigen preferably comprises several T-cell epitopes, which is often the case if an antigen comprising more than 20 aa, more than 25 aa, more than 50 aa or more than 100 aa is used. The length of one T-cell epitope typically is 8-10 aa. Methods of identifying T-cell epitopes are known to the skilled person. Use of a complete surface protein of a pathogen targeted by the vaccination or at least the extracellular part thereof (or the part that is accessible to antibodies on the surface of a pathogen) is also advantageous for this reason. It also minimizes the risk of immune evasion by mutation of the pathogen. The same applies for cancer antigens that can also be the target of vaccination.

The antigen typically is a polypeptide or comprises the same. It can also be a carbohydrate, or it can comprise a carbohydrate, e.g., a polysaccharide. The term polysaccharide is understood to comprise oligosaccharides herein. In one embodiment, the antigen is not a fluorescent marker such as FITC, Cy5, and it is not dinitrophenyl (DNP), dextran sulphate or 2,4-dichlorophenoxyacetic acid (2,4-D). In one embodiment, it is also not a small molecule (organic molecule having a molecular weight of up to 800 Da that is not a biopolymer such as a peptide or polysaccharide).

The antigen comprised in the conjugate, to which the subject is to be vaccinated, can e.g., be a peptide comprising or consisting of SEQ ID NO: 1 or 2, i.e., a SARS-CoV-2 derived peptide, if the vaccination is against SARS-CoV-2. The antigen can also comprise SARS-CoV-2 spike protein or the part thereof present on the surface of a viral particle. In one embodiment, the IgA- or IgM binding agent of the conjugate is derived from a SARS-CoV-2 spike antigen of a variant to which there is a high prevalence of IgA-mediated immunity in the population of interest, and the antigen is a SARS-CoV-2 derived peptide from a new variant to which immunity is to be induced. Thus, the vaccination can build upon pre-existing immunity to older variants of a pathogen.

The conjugate comprised in the invention can alternatively comprise a nucleic acid, e.g., DNA or RNA encoding an antigen, e.g., an antigen as described herein. The nucleic acid typically is RNA, e.g., mRNA, typically, in a form comprising modified nucleotides and/or structures that makes the RNA more stable than classical RNA. Suitable RNA forms that can be used for vaccination are known in the art.

The invention also provides a vaccine that can be used for mucosal vaccination, as such, in particular, a vaccine that comprises an agent capable of specifically binding to the immunoglobulin independently from the paratope of the immunoglobulin, as described herein. Accordingly, one object of the invention is a vaccine comprising a conjugate of
a) an agent capable of specifically binding to an immunoglobulin, wherein the immunoglobulin is IgA or IgM, and
b) an antigen or a nucleic acid, e.g., RNA, encoding an antigen,
wherein the vaccine does not comprise IgA or IgM,
and wherein the vaccine preferably does not comprise an adjuvant. Preferably, the agent is capable of specifically binding to the immunoglobulin independently from the paratope of the immunoglobulin, e.g., it can be an IgA-binding peptide, an IgA-binding peptidomimetic, an IgA-binding small molecule, such as an IgA-binding nucleotide / nucleotide mimetic or a conjugate of both. The definitions explained herein may also apply to the vaccine.

The invention also provides a method for mucosal vaccination of a subject against an antigen, comprising mucosally administering an effective amount of a vaccine comprising a conjugate of
a) an agent capable of specifically binding to an immunoglobulin, wherein the immunoglobulin is IgA or IgM, and
b) the antigen or a nucleic acid, e.g., RNA, encoding the antigen
to a subject, wherein the vaccine does not comprise IgA or IgM, and, preferably, wherein the vaccine does not comprise an adjuvant.

The vaccine of the present invention is for use in mucosal administration. Accordingly, the vaccine may be for intranasal administration, for intrapulmonal administration, for gastrointestinal administration, for oral administration, or for vaginal administration. Transmucosal administration may also be administration to an oral mucosa, e.g. sublingual administration or buccal administration. Preferably, the vaccine is to be administered to the airways of the subject, most preferably, by intranasal administration.

The preferred site of administration may be selected depending on the site at which an induction of a mucosal immune response is particularly desired, e.g., a vaccination against an airborne pathogen may preferably be a nasal or intrapulmonal administration, or a vaccination against a gastrointestinal pathogen may preferably be an oral or gastrointestinal administration. However, there is immunological crosstalk between different mucosal sites, and mucosal immunity may also be induced for sites where the vaccine is not administered, so site-specific administration may not be required.

The site of administration in the gastrointestinal tract may e.g. be selected due to encapsulation. Methods for formulating an active agent in a way that allows for stable passage through the stomach and/or release in a desired zone of the gastrointestinal tract are well known to the skilled person. For example, the vaccine may be administered to an airway of the subject or to the oral mucosa in the form of a spray, a solution suitable for mucosal administration (e.g., as nose drops) or by inhalation. Administration to the airway such as nasal administration is generally preferred, as peptides are typically more rapidly degraded in the gastrointestinal tract. Further, the mucosal immune system of the airways ages more slowly than the gastrointestinal immune system.

The vaccine may comprise a solvent, e.g., a buffer and/or a liquid excipient. The solvent typically is a water-based solvent, e.g., a buffer, which may or may not comprise a preservative. It may also comprise ethanol and/or other organic solvents such as DMSO, optionally, in a mixture with water. The vaccine can be formulated, e.g., buffered and/or stabilized, according to methods known in the art. For examples, liposomal vesicles may be used. However, as liposomes are typically useful for mediating cell entry, and this is otherwise mediated in the context of the present invention, liposomes are not required. Advantageously, none of the components of the vaccine however is supposed to act as an adjuvant, i.e., in case liposomes are used, preferably, they should not contain components that stimulate the immune system. An adjuvant in the context of the invention is an immunological adjuvant that can stimulate the immune system in an antigen-independent manner, in particular through mimicking sets of evolutionarily conserved molecules, so-called pathogen-associated molecular patterns, which include liposomes, lipopolysaccharide, molecular cages for antigens, components of bacterial cell walls, and nucleic acids such as RNA, double-stranded RNA, single-stranded DNA, and unmethylated CpG dinucleotide-containing DNA.

Specifically, preferably, the vaccine does not comprise inorganic compounds that are used as adjuvants, namely, potassium alum, aluminum hydroxide, aluminum phosphate or calcium phosphate hydroxide, oils, such as paraffin oil, propolis, Adjuvant 65 (based on peanut oil), bacterial products having an adjuvant effect, such as killed bacteria of the species *Bordetella pertussis*, *Mycobacterium bovis* toxoid, Cholera toxoid, flagellin, or LPS. It may comprise bacterial products not capable of stimulating the immune system (e.g., stimulating professional antigen-presenting cells in a non-antigen-specific manner) as such. The vaccine also preferably does not comprise plant saponins, e.g., from Quillaja (see Quil A), soybean, or Polygala senega or cytokines, e.g., IL-1, IL-2, or IL-12, Freund's complete adjuvant, Freund's incomplete adjuvant, or squalene.

In a particularly preferable embodiment, the vaccine does not comprise an active agent separate and additional to a) the IgA binding agent and b) the antigen defined above. Thus, in this embodiment, the antigen to which the subject is to be immunized may have an adjuvant effect (e.g., in the context of a mucosal vaccination to cholera using cholera toxin B as the antigen in the conjugate of the invention), but, preferably, there is no additional adjuvant in the vaccine.

The vaccine can advantageously be used for vaccination against a mucosal pathogen. Said pathogen may be, e.g., influenza virus, SARS-CoV-2, HIV, SARS-CoV-1, MERS, RSV, poliovirus, rotavirus, hepatitis C virus, papillomavirus, herpes simplex virus, human rhinovirus, Zika virus, hanta virus, nipah virus, mpox virus, *Corynebacterium (e.g., C. diphteriae), Mycobacterium (e.g., M. tuberculosis, M. leprae or M. bovis), Bordatella (e.g., B. pertussis), Brucella (e.g., B. melitensis), Chlamydia (e.g., C. trachomatis), Streptococcus (e.g., S. pneumoniae), Haemophilus (e.g., H. influenzae), Helicobacter (e.g., H. pylori), enterotoxigenic Escherichia coli, Salmonella (e.g., S. typhimurium or S. enterica), Vibrio (e.g., V. cholerae), Shigella (e.g., Sh. dysenteriae or Sh. flexneri),* and *Entamoeba (e.g., E. histolytica)* or against a cancer selected from the group comprising lung cancer, stomach cancer, colorectal cancer, cervical cancer and ovarian cancer. The antigen of the conjugate comprised in the vaccine at least comprises a B cell antigen of said pathogen or cancer, or it is a nucleic acid encoding the same.
**Fig. 1****:** Principle of mucosal vaccination via IgA feedback loop: **A:** Mucosal IgA feedback loop: slgA patrolling the lumen are re-internalised via the mucosal epithelia and transported into the submucosal lymphatic tissue. If IgA has bound an antigen, this is an immunostimulatory signal, which leads to production of more IgA against this antigen. **B, C:** Concepts for exploiting the IgA feedback loop: The antigen of interest (Y) is coupled to an IgA binder. After internalisation of the IgA binder-antigen-conjugate that forms the vaccine, secretion of IgA against the antigen is also induced. **B**: As IgA binder, an antigen (X) is selected against which the subject to be vaccinated has a pre-existing slgA immunity, such that a binding to the corresponding paratope of the antibody can occur. **C**: Alternatively, a generic IgA binder (BA) is used that binds IgA independently of the antigen binding site of the antibody, e.g. that binds to the Fc region of IgA. Alternatively, an IgM binder can be used.
**Fig. 2****:** SDS-polyacrylamide gel electrophoretic analysis of conjugates between carrier protein and peptide antigens. Left: OVA & SARS-CoV-2-spike Wuhan peptide; right: OVA & SARS-CoV-2-spike omicron peptide. Lane M: size standard, lane 1: OVA before conjugation, lane 2: OVA-SARS vaccination conjugate after dialysis

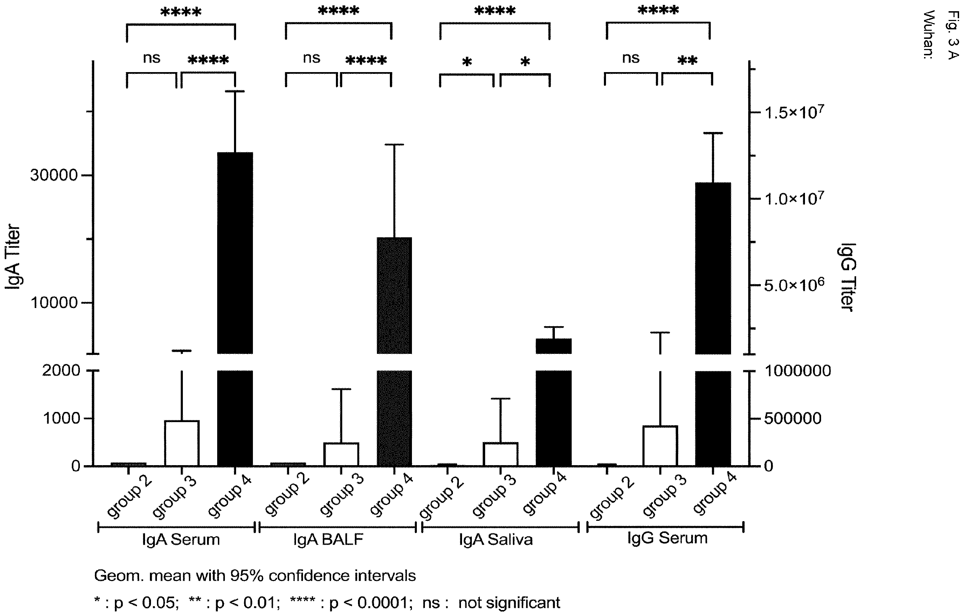

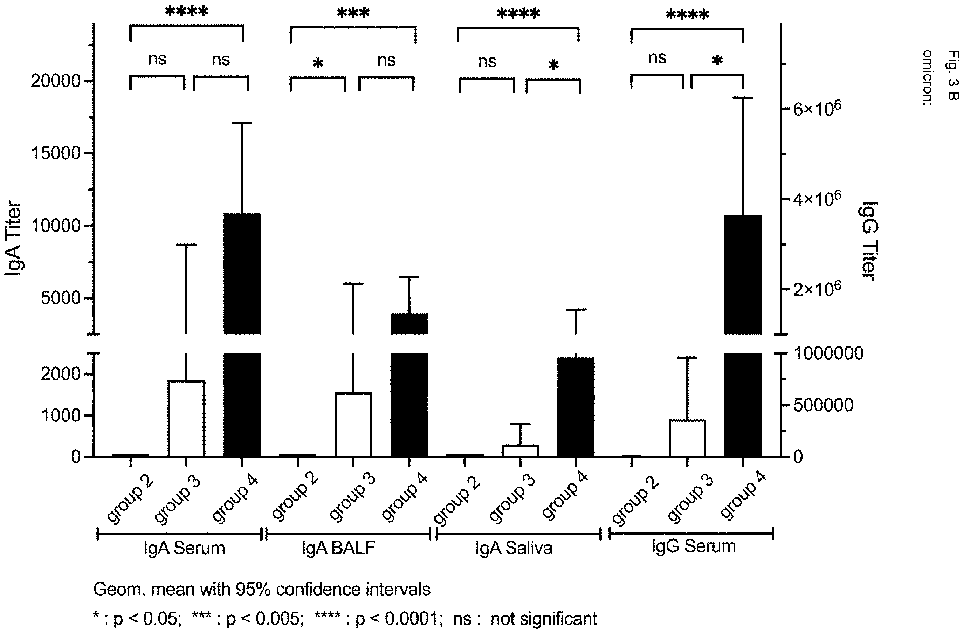

**Fig. 3****:** Anti-SARS-CoV-2 spike peptide endpoint IgA- and IgG titers in saliva, broncho-alveolar lavage fluid (BALF) and serum of mice receiving the OVA-SARS-vaccine conjugate following different immunization protocols. Geometric means and 95% confidence intervals are shown. Between-group comparisons were done using a Kruskal-Wallis test with Dunn's multiple comparisons as post hoc analysis. **A** SARS-CoV-2 alpha **B** SARS-CoV-2 omicron
**Fig.4****:** IgA binding capability of peptides generated in a molecular evolution process. The EC₅₀ values for IgA binding for the top performing peptides of the lead population (SEQ ID NO: 29-31) and each filial generation are shown (SEQ ID NO: 35-47). The two evolved peptides with highest IgA affinity (EC₅₀ = 0.9 µM) are symbolized by black bars.

### EXAMPLES

### 1. Induction of antigen-specific IgA by intranasal immunization with an antigen-IgA paratope binder conjugate, utilizing preexisting anti-IgA binder IgA-immunity

An experimental mucosal immunization employing the method proposed in the present invention was carried out in Balb/c mice. As there is no defined pre-existing immunity in these mice, a two-step protocol was used: For this approach, experimental animals received first a conventional intranasal immunization with a protein chosen to serve as IgA-paratope binder (ovalbumin, OVA) in the presence of cholera toxin (CT) as mucosal adjuvant to generate an anti-OVA IgA immunity (stage 1 of the immunization). In stage 2 of the immunization, the animals received repeated intranasal applications of a vaccination conjugate consisting of a peptidic antigen coupled to OVA (i.e., the agent capable of specifically binding to the paratope of IgA induced in the first stage of the immunization), in the absence of mucosal adjuvant. As vaccination antigens, two 20mer peptides from the receptor binding domain of the SARS-CoV-2 spike protein (corresponding to amino acids 490-509 of SARS-CoV-2 spike S1, numbering based on Wuhan variant) were chosen. Control experiments included (i) intranasal immunization of mice with adjuvant only, (ii) intranasal immunization of mice with the vaccination conjugate in the absence of adjuvant, without prior generation of anti-OVA IgA immunity, and (iii) intranasal immunization of mice with the vaccination conjugate in the presence of adjuvant (Table 1).

The outcome of the immunizations was evaluated by measuring the antigen-specific IgA response in saliva and bronchoalveolar fluid (BALF), as well as the IgG and IgA response in serum.

**Table 1: Immunization groups with different antigen combinations**

| Group | | Vaccine Stage 1 | Adjuvant Stage 1 | Vaccine Stage 2 | Adjuvant Stage 2 |
|---|---|---|---|---|---|
| 1 | Control (7 animals) | - | CT | - | - |
| 2 | Control (7 animals) | - | CT | OVA-SARS-conjugate | - |
| 3 | Control (7 animals) | OVA-SARS-conjugate | CT | - | - |
| 4 | Experiment (14 animals) | OVA | CT | OVA-SARS-conjugate | - |

### Synthesis of vaccination conjugates

The SARS-CoV-2 spike-peptides (Wuhan: FPLQSYGFQPTNGVGYQPYR (SEQ ID NO: 1); omicron: FPLKSYSFRPTYGVGHQPYR (SEQ ID NO: 2)) were synthesized by Fmoc solid phase peptide synthesis on a TentaGel S RAM resin (0.26 mmol/g, Rapp Polymere, Tübingen, DE) in 30 µmole scale using an automated peptide synthesizer (CEM MultiPep RS, Cologne, DE) along with commercially available amino acid building blocks (Iris Biotech, Marktredwitz, DE). Peptides were extended with a cysteine at their carboxy terminus for subsequent coupling to carrier proteins. After cleavage from the synthesis resin and side chain deprotection, using 92.5% (v/v) trifluoroacetic acid, 5% (v/v) triisopropylsilane, 2.5% (v/v) water, peptides were precipitated using 50% (v/v) tert.-butyl-methyl-ether / cyclohexane and subsequently lyophilized from 20% (v/v) acetonitrile / water containing 1% (v/v) acetic acid. Identity and purity (>90%) of the peptides was confirmed by HPLC/MS analysis.

To obtain the vaccination conjugate, peptides were coupled to hen egg white ovalbumin (OVA). 500 µl of 20 mg/ml OVA in Dulbecco's phosphate buffered saline (D-PBS;10 mM Na₂HPO₄, 1 mM KH₂PO₄, 137 mM NaCl, 2.7 mM KCI) were cooled to 4°C, 5.3 µl of 100 mg/ml bromoacetic acid N-hydroxysuccinimide (NHS-BrAc) in DMF were added, and the mixture was incubated under slight agitation for 30 min in the dark, thereby reaching room temperature. After cooling the reaction mixture again to 4°C, addition of NHS-BrAc and incubation was repeated once, and the reaction was stopped by adding 150 µl of 3 M glycine in D-PBS and incubation for 45 min at room temperature in the dark. The reaction mixture was dialyzed against D-PBS for 24 h in the dark. The preactivated OVA was added to 5.5 mg of SARS-CoV-2 spike-peptide dissolved in 20 µl DMF (approximately 10-fold molar excess of peptide over OVA), and the reaction mixture was incubated under slight agitation for 4 h in the dark at room temperature.

The reaction was stopped by adding 45 µl of 1 M cysteine in D-PBS and incubation for 90 min at room temperature in the dark. Unconjugated peptide was removed by dialysis against D-PBS for 48 h. Conjugation of peptide molecules to OVA was verified by SDS polyacrylamide gel electrophoresis (Fig. 2).

### Immunization & sampling procedures

Balb/c mice aged 7 weeks were randomly assigned to 4 groups according to Table 1. Three days prior to the first immunization, blood and saliva samples were collected.

For immunization, animals were sedated by placing them into a saturated sevoflurane (Ecuphar, Greifswald, DE) atmosphere. As soon as the mice were asleep, a micropipet was used to apply 20 µl of vaccine solution (20 µg antigen ± 1 µg cholera toxin adjuvant (List Labs, Campbell, CA, USA) in D-PBS) onto the nostrils. The treatment schedule and immunization scheme for all groups is given in Table 2.

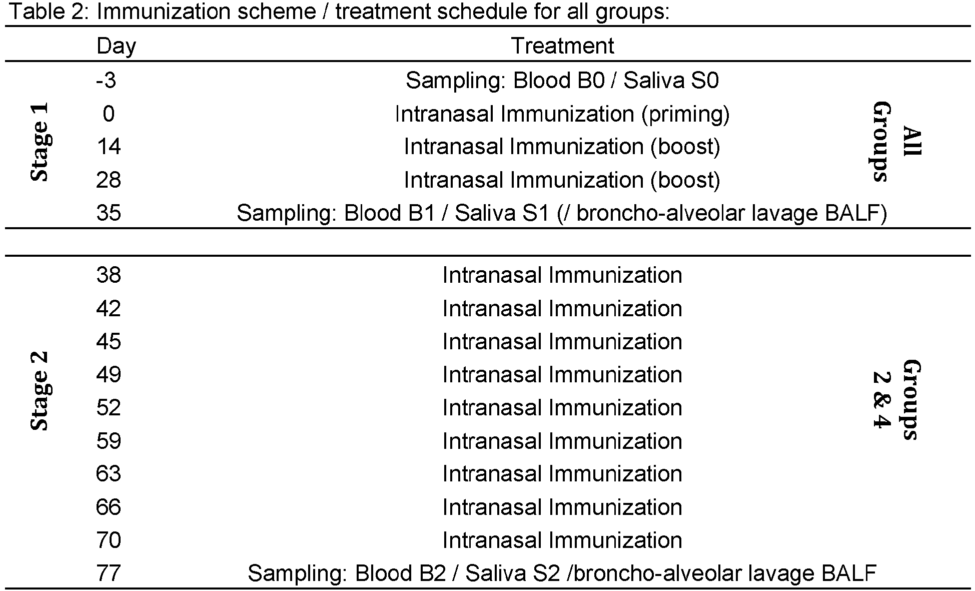

To collect saliva samples at the beginning and end of stage 1, animals were anesthetized by i.p. injection of 100 mg/kg ketamine / 10 mg/kg xylazine in 0.9% NaCl. To induce saliva production, 40 µl of 100 µg/ml pilocarpine in 0.9% NaCl were injected intraperitoneally, and saliva was collected from nostrils and mouth with a micropipet; blood was collected from small punctures in the tail vein. Blood was allowed to clot overnight at room temperature followed by incubation at 4 °C for 1h. Afterwards the blood was centrifuged at 16,100 x g for 15 min at 4 °C to obtain the sera.

At the end of the study (end of stage 1 for groups 1 & 3, end of stage 2 for groups 2 & 4), saliva was collected after anesthetization and pilocarpine treatment as described above. Afterwards, animals were sacrificed by i.p. injection of 180 mg/kg ketamine / 15 mg/kg xylazine in 0.9% NaCl, the thorax was opened, and blood was collected. The trachea was opened with a small incision, and BALF was obtained by injecting and removing 1 ml of D-PBS.

### ELISA

SARS-CoV-2 spike peptides were conjugated to myoglobin (MYO) as carrier protein using bromoacetic acid N-hydroxysuccinimide as described above; unconjugated peptide was removed by dialysis against D-PBS, the conjugate (MYO-SARS-conjugate) was used as coating antigen in ELISA.

To determine specific anti-SARS-peptide or anti-ovalbumin antibody titers, 96-well high-bind polystyrene plates (Costar) were coated with 75 µl/well of 2 µg/ml MYO-SARS-conjugate in D-PBS or 5 µg/ml OVA in 50 mM sodium acetate buffer, pH 5.0, overnight at 4°C. Plates were washed 3 times with 300 µl/well of D-PBS/0.02% (v/v) Tween20 (PBS-T) using an automated plate washer. For blocking, all wells were filled with 300 µl/well of blocking solution and incubated for 5-6 h at room temperature. Blocking solution was 1% (w/v) Gifraxil OD 20 M (Industria Chimica Panzeri, Bergamo, Italy) in D-PBS for ELISA analyzing immunoglobulin reactivity against SARS-CoV-2 spike Wuhan peptide, or 1% (w/v) non-fat dry milk in D-PBS for all other ELISA. Plates were washed 4 times with 300 µl/well of PBS-T, and 75 µl/well of serum / saliva / BALF sample (two-fold serial dilutions in blocking buffer) were added. Plates were incubated over night at 4°C and washed 4 times with 300 µl/well of PBS-T. 75 µl/well of goat anti-mouse IgA-HRP (Southern Biotech) or goat anti-mouse IgG-HRP (Southern Biotech), diluted 1:2.000 in blocking buffer, were added and incubated for 90 min at room temperature. Plates were washed 6 times with 300 µl/well of PBS-T, before 75 µl/well of TMB substrate solution (Frey et al., 2000) were added. Plates were incubated for 30 min at room temperature, 125 µl/well 1 M H₂SO₄ were added, and absorbance at 450 nm was measured using a microplate reader (Spectramax M5 running SoftMaxPro v.5.3, Molecular Devices, Sunnyvale, CA, USA).

Endpoint titers against the respective antigens in serum, saliva and BALF samples were determined according to the method of Frey et al. (1998). Corresponding samples obtained from animals in control group 1 were used to calculate the cut-off value for a confidence level of 99.5%.

Statistical analyses were performed using GraphPad Prism v 9.5.1 (GraphPad, Boston, MA, USA); between-group comparisons were done using a Kruskal-Wallis test with Dunn's multiple comparisons as post hoc analysis.

### Results

Conjugation of the SARS-CoV-2 spike Wuhan and SARS-CoV-2 spike omicron peptide to the carrier protein yielded conjugates of ovalbumin (44.3 kDa) carrying 1 to 3 peptide molecules (2.5 kDa), as estimated from band shift in Coomassie-stained SDS-polyacrylamide gels (Fig. 2).

The OVA-SARS-CoV-2 spike peptide conjugates were used in two independent immunization experiments, one employing a SARS-CoV-2 spike peptide of the Wuhan variant (SEQ ID NO: 1), the second employing the corresponding peptide taken from the omicron variant (SEQ ID NO: 2). In both immunizations, anti-OVA IgA were first (stage 1) induced by intranasal immunization with ovalbumin in the presence of cholera toxin adjuvant in those mice intended for immunization according to the method proposed in the current invention (group 4). Control mice were given cholera toxin only, without ovalbumin. After three applications of the vaccine formulation following the immunization scheme given in Table 2, strong anti-OVA responses were attained in all mice belonging to experimental group 4, while no anti-OVA IgA were detectable in mice of group 2 (Table 3).

**Table 3: Anti-ovalbumin IgA endpoint titers in saliva of mice after immunization stage 1:**

| Immunization experiment | SARS-CoV-2 spike Wuhan | | SARS-CoV-2 spike omicron | |
|---|---|---|---|---|
| Group | 2 | 4 | 2 | 4 |
| anti-OVA IgA titer (geom. mean) | n.d. | 16,320 | n.d. | 4,714 |

| | | | | |
|---|---|---|---|---|
| n.d.: not detectable | | | | |

In stage 2 of the immunization, the vaccine conjugate of ovalbumin and SARS-CoV-2 spike peptide was given intranasally, in the absence of adjuvant. After a total of 9 applications over a period of 5 weeks, all mice in group 4 showed strong anti-SARS-CoV-2 spike peptide immune responses in serum as well as in mucosal secretions (Fig. 3). Application of the vaccine conjugate without adjuvant to mice without pre-existing anti-OVA immunity (group 2) led to weak anti-SARS-CoV-2 spike peptide IgG responses in serum of few animals, no specific IgA reactivity was detected in either serum, saliva or BALF. "Positive" control immunizations with vaccine conjugate in the presence of cholera toxin adjuvant (group 3) led, as expected, to anti-SARS-CoV-2 spike peptide immune responses in serum and mucosal secretions. Most surprisingly, the IgA and IgG titers achieved with the immunization method proposed in the current invention was in all cases higher than the titers achieved with the standard mucosal immunization protocol employing a mucosal adjuvant (geometric means of endpoint titers of groups 3 versus groups 4 in both immunizations; Fig. 3).

### 2. Evolution of improved IgA-binding peptides

A genetic algorithm-based molecular evolution procedure was used to generate peptides with improved IgA-binding properties as compared to peptides disclosed by Ito et al. in e.g. WO 2013/081037 A1. The evolution procedure started with peptides of SEQ ID NO: 5, 25, 27 and 29-31, and it was performed according to the protocol developed by Röckendorf et al. (2012, PloS Comput. Biol. 8:e1002800) and described in detail in Röckendorf et al. (2022, Methods Mol. Biol. 2383:45). Peptides were ranked and underwent further optimization with respect to their IgA binding capability as determined as EC₅₀ value in an IgA binding assay.

### Molecular evolution

The genetic algorithm software was applied to a starting population of six 18mer peptides, taken from Hatanaka et al. (2012), and a filial generation of 36 peptide sequences was computed, utilizing a mutation rate setting of 12%. In all generations, positions 4 (cysteine), 7 (tyrosine) and 13 (cysteine) were kept constant. The resulting peptides were synthesized by Fmoc solid phase peptide synthesis on biotin-PEG-Nova tag resin (Merck, Darmstadt, Germany) using an automated peptide synthesizer along with commercially available amino acid building blocks as described above. After cleavage from the resin, the precipitated peptides - each carrying a carboxy terminal PEG linker and a biotin tag - were dissolved in NH₃/water (pH ~7.3), and analyzed by HPLC/MS. Cyclization occurred spontaneously or was induced by stirring in open vials for 12 h at room temperature. Concentrations of peptide preparations were determined photometrically at 280 nm. Peptides were analyzed for their IgA-binding capability, and their performance in this assay, expressed as the EC₅₀-value, was entered as fitness value into the genetic algorithm for computing the next generation of peptide sequences. The top 8 candidate peptides of each generation's sequence pool were selected as parent peptides for the next generation.

### Solid-phase IgA-binding assay

IgA binding capability of IgA binding peptides or IgA binding agents can be determined as EC₅₀ value in a solid-phase IgA binding assay, wherein the peptides or agents of interest (optionally, conjugated via biotin to streptavidin or avidin to optimize coating) are coated on a solid support, and, after blocking, serial dilutions of secretory IgA, e.g., from human colostrum, are contacted to the support, and, after washing, binding of the IgA is detected.

All cyclic peptides were diluted in D-PBS to yield 10 µM solutions. 45 µl of these solutions were incubated with 5 µl of 1 mg/mL streptavidin (Vector Laboratories, Newark, CA, USA) for 6 h at room temperature. Excess peptide was removed by dialysis, streptavidin-peptide conjugates were further diluted to a final concentration of 1 µg/mL in D-PBS and were used to coat high-binding polystyrene microtiter plates using 75 µL/well in D-PBS for 15 h at 4°C. After washing the plates three times with 300 µl/well of PBS-T, plates were blocked for 2 h at room temperature with 250 µl/well of a solution of 1% w/v of CO/15 (Industria Chimica Panzeri, Bergamo, IT) in D-PBS (blocking solution). The plates were washed four times with 300 µl/well of PBS-T, and 75 µL/well of secretory IgA from human colostrum (MP Biomedicals, Eschwege, DE) in blocking solution were applied in two-fold serial dilutions. The plates were washed four times with 300 µl/well of PBS-T and subsequently incubated with 75 µL/well of sheep anti-human IgA-HRP (The Binding Site, Birmingham, AL, US), diluted 1:2,000 in blocking solution. The plates were incubated for 120 min at room temperature. After washing the plates six times with 300 µL/well of PBS-T, 75 µL/well of TMB substrate solution (Frey et al., 2000) were added. After 30 min incubation at room temperature, the reaction was stopped by adding 125 µL/well of 1 M H₂SO₄. Optical densities at 450 nm were determined on a microplate reader (Spectramax M5 running SoftMaxPro v.5.3, Molecular Devices, Sunnyvale, CA, USA). Sigmoidal titration curves were generated and EC₅₀ values were calculated using GraphPad Prism v 10.0 (GraphPad, Boston, MA, USA).

### Results

A total of 3 filial generations encompassing 36 peptides each was computed, synthesized, and analyzed. For each peptide, the sigmoidal curves for IgA binding were interpolated from the A₄₅₀ absorbance values versus IgA concentration, and EC₅₀ values were calculated. The EC₅₀ values of the six IgA binding peptide candidates used as the lead peptide population ranged from 1.6 µM to 10.2 µM in this assay set-up. In each of the following generations, peptides with increased IgA affinity, i.e. lower EC₅₀ value, were obtained (Fig. 4). The top two peptides KMVCLAYR-GRPVCFPT (SEQ ID NO 35) and HSVCLAYRGRPVCFPT (SEQ ID NO 36) had an EC₅₀ value each of 0.9 µM, thereby exhibiting a higher IgA affinity than any of the lead peptides taken from the literature (Hatanaka et al., 2012).

### References

Holmgren, J., Svennerholm, A.-M. (2012) Vaccines against mucosal infections. Curr Opin. Immunol. 24:343-353.
Baker, J.R., Farazuddin, M., Wong, P.T., O'Konek, J.J. (2022) The unfulfilled potential of mucosal immunization. J. Allergy Clin. Immunol. 150:1-11
Topol, E.J., Iwasaki, A. (2022) Operation nasal vaccine - lightning speed to counter COVID-19. Sci. Immunol. 7:eadd9947
Correa, V.A., Portilho, A.I., De Gaspari, E. (2921) Vaccines, adjuvants and key factors for mucosal immune response. Immunology 167:124-138.
Lavelle, E.C., Ward, R.W. (2022) Mucosal vaccines - fortifying the frontiers. Nat. Rev. Immunol. 22:236-250
Tate J.E., Parashar, U.D. (2014) Rotavirus vaccines in routine use. Vaccines 59:1291
Troeger, C., et al. (2018) Rotavirus vaccination and the burden of rotavirus diarrhea among children younger than 5 years. JAMA Pediatr. 172:958-965.
Burns, C.C., Diop, O.M., Sutter, R.W., Kew, O.M. (2014) Vaccine-derived polioviruses. J. Infect. Dis. 210S1:S283-S293.
Roberts, L. (2018) Polio outbreaks in the DRC threaten eradication effort. Science 361:10-11 Frey, A., Meckelein, B., Externest, D. Schmidt, M.A. (2000) A stable and highly sensitive 3,3',5,5'-tetramethylbenzidine-based substrate reagent for enzyme-linked immunosorbent assays. J. Immunol. Methods 233:47-56
Frey, A., Di Canzio, J., Zurakowski, D. (1998) A statistically defined endpoint titer determination method for immunoassays. J. Immunol. Methods 221:35-41
Corthésy, B., Kaufmann M., Phalipon A., Peitsch M., Neutra M.R., Kraehenbuhl J. (1996) A pathogen-specific epitope inserted into recombinant secretory immunoglobulin A is immunogenic by the oral route. Journal of Biological Chemistry 271:33670-33677.
Crottet et al. (1999) Covalent homodimers of murine secretory component induced by epitope substitution unravel the capacity of the polymeric Ig receptor to dimerize noncovalently in the absence of IgA ligand. Journal of Biological Chemistry 274:31445-31455.
Rochereau N, Drocourt D, Perouzel E, Pavot V, Redelinghuys P, et al. (2013) Dectin-1 is essential for reverse transcytosis of glycosylated slgA-antigen complexes by intestinal M cells. PLoS Biol 11(9): e1001658. doi:10.1371/journal.pbio.1001658
Rochereau N. et al. (2015). Secretory IgA as a vaccine carrier for delivery of HIV antigen to M cells Eur. J. Immunol. 45: 773-779
Rochereau N. et al. (2016). Delivery of antigen to nasal-associated lymphoid tissue microfold cells through secretory IgA targeting local dendritic cells confers protective immunity. J Allergy Clin Immunol 137:214-22.
Rochereau N. et al. (2021) Essential role of TOSO/FAIM3 in intestinal IgM reverse transcytosis. Cell Reports 37, 110006.
Fransen et al. (2015) BALB/c and C57BL/6 mice differ in polyreactive IgA abundance, which impacts the generation of antigen-specific IgA and microbiota diversity, Immunity, http://dx.doi.org/10.1016/j.immuni.2015.08.011.
Corthésy, B., Bioley G. (2018). Lipid-based particles: versatile delivery systems for mucosal vaccination against infection. Frontiers in Immunology 9: 431, doi: 10.3389/fmmu.2018.00431.
Szoka (2022). A hitchhiker's guide to mucosal and systemic immunity. Sci. Transl. Med. 14, eadc8697
Hartwell B.L. et al. (2022). Intranasal vaccination with lipid-conjugated immunogens promotes antigen transmucosal uptake to drive mucosal and systemic immunity. Sci. Transl. Med. 14, eabn1413.
Liu H. et al. (2014). Structure-based programming of lymph-node targeting in molecular vaccines. Nature 507:519-522
Yang D. et al. (2016) Comparison of biosensor platforms in the evaluation of high affinity antibody-antigen binding kinetics Anal. Biochem. 508, 78-96
Kirley T.L. & Norman A.B. (2022) Isothermal titration calorimetry determination of thermodynamics of binding of cocaine and its metabolites to humanized h2E2 anti-cocaine mAb. Biochem. Biophys. Rep. 32, 101354
Rajagopalan K. et al. (1996) Novel unconventional binding site in the variable region of immunoglobulins. Proc. Natl. Acad. Sci. USA 93, 6019-6024
Alves N. et al. (2012) Small-molecule-based affinity chromatography method for antibody purification via nucleotide binding site targeting. Anal. Chem. 84, 7721-7728
Johnsson E. et al. (1999) An IgA-binding peptide derived from a Streptococcal surface protein. J. Biol. Chem. 274:14521-14524
Röckendorf N. et al. (2012) Molecular evolution of peptide ligands with custom-tailored characteristics for targeting of glycostructures. PloS Comput. Biol. 12:e1002800
Röckendorf N. et al. (2022) Artificial evolutionary evolution processed to improve the functionality of cell penetrating peptides. Methods Mol. Biol. 2383:45-61.
Hatanaka T. et al. (2012) Human IgA-binding peptides selected from random peptide libraries. J. Biol. Chem. 287:43126-43136.
EP 0 839 915 A1, WO 98/20141 A1, US 2022112276 A1, WO 2009/156307 A1
WO 2011/148952 A1
WO 2013/081037 A1
WO 1984/001294 A1 or WO 2003/080655 A1
WO 2000/063383 A1

## Claims

1. A vaccine comprising a conjugate of
a) an agent capable of specifically binding to an immunoglobulin, wherein the immunoglobulin is IgA or IgM, and
b) an antigen or a nucleic acid encoding an antigen,
for use in mucosal vaccination of a subject against said antigen, wherein the vaccine does not comprise IgA or IgM.

2. The vaccine for use of claim 1, wherein the immunoglobulin is IgA, preferably, slgA.

3. The vaccine for use of claim 1, wherein the immunoglobulin is IgM, preferably, slgM.

4. The vaccine for use of any of claims 1-3, wherein the agent is capable of specifically binding to the immunoglobulin independently from the paratope of the immunoglobulin, preferably, wherein the agent is capable of specifically binding to a constant part of the immunoglobulin, optionally, to a constant part of slgA.

5. The vaccine for use of claim 4, wherein the agent is an IgA binder comprising an IgA binding peptide, optionally, an IgA binding peptide having any of SEQ ID NO: 3-47.

6. The vaccine for use of claim 4, wherein the agent is an IgA binder comprising an anti-lgA antibody or a fragment of said antibody that comprises an Fv region, preferably, an scFv.

7. The vaccine for use of claim 4, wherein the agent comprises an IgA receptor selected from the group comprising polymeric Ig receptor, myeloid specific IgA Fc receptor (FcαRI), Fcα/µR, asialoglycoprotein receptor, M-cell IgA receptor and transferrin receptor or a fragment of said IgA receptor capable of binding the Fc part of IgA.

8. The vaccine for use of claim 4, wherein the agent is an IgM binder selected from the group comprising protein A, FcµR, and Fcα/µR.

9. The vaccine for use of claim 4, wherein the agent is a small molecule having up to 800 Da, optionally, wherein the small molecule is a nucleotide or a nucleotide analogue/mimetic.

10. The vaccine for use of any of claims 1-3, wherein the agent is capable of specifically binding to the paratope of pre-existing IgA or IgM in the subject.

11. The vaccine for use of claim 10, wherein the agent comprises a biopolymer comprising at least one B-cell epitope, preferably, a whole polypeptide, wherein the polypeptide optionally is a SARS-CoV-2 spike antigen, an influenza HA, a *Streptococcus pneumoniae* antigen, a rotavirus antigen, a rhinovirus antigen, or an antigen of a commensal bacterial species selected from the group comprising Lactobacillus, Bifidobacterium, Bacteroides, and Escherichia.

12. The vaccine for use of any of the preceding claims, wherein the vaccine does not comprise an adjuvant.

13. The vaccine for use of any of the preceding claims, wherein subject is human and the mucosal immunoglobulin is human.

14. The vaccine for use of any of the preceding claims, wherein the vaccine is for intranasal administration, for intrapulmonal administration, for oral administration, gastrointestinal administration or vaginal administration, preferably, for administration to the airways of the subject, most preferably, for intranasal administration.

15. The vaccine for use of any of the preceding claims, wherein the vaccination is vaccination against a mucosal pathogen selected from the group comprising influenza virus, SARS-CoV-2, HIV, SARS-CoV-1, MERS, RSV, poliovirus, rotavirus, hepatitis C virus, papillomavirus, herpes simplex virus, human rhinovirus, Zika virus, hanta virus, nipah virus, mpox virus*, Corynebacterium, Mycobacterium, Bordatella, Brucella, Chlamydia, Streptococcus, Haemophilus, Helicobacter, enterotoxigenic Escherichia coli, Salmonella, Vibrio, Shigella,* and *Entamoeba* or against a cancer selected from the group comprising lung cancer, stomach cancer, colorectal cancer, cervical cancer and ovarian cancer.

16. An IgA binding peptide having any of SEQ ID NO: 32-34, wherein amino acid position 15 of the IgA-binding peptide is P and amino acid position 15 of the IgA-binding peptide is T, or any of SEQ ID NO: 35 and 47.
